## Europäisches Patentamt
## European Patent Office
## Office européen des brevets

(19)

(11) Veröffentlichungsnummer: **0 220 478**
**B1**

(12) **EUROPÄISCHE PATENTSCHRIFT**

(45) Veröffentlichungstag der Patentschrift:
**13.09.89**

(51) Int. Cl.⁴: **A61F 7/00,** F25B 19/00,
F25D 3/10

(21) Anmeldenummer: **86112926.0**

(22) Anmeldetag: **18.09.86**

(54) **Vorrichtung zur Durchführung der Kryotherapie.**

(30) Priorität: **28.09.85 DE 3534629**

(43) Veröffentlichungstag der Anmeldung:
**06.05.87 Patentblatt 87/19**

(45) Bekanntmachung des Hinweises auf die Patenterteilung:
**13.09.89 Patentblatt 89/37**

(84) Benannte Vertragsstaaten:
**AT BE CH FR GB IT LI NL SE**

(56) Entgegenhaltungen:
**AT-B- 376 032**
**DE-A- 3 242 881**
**US-A- 3 885 571**

(73) Patentinhaber: **MESSER GRIESHEIM GMBH, Hanauer
Landstrasse 330, D-6000 Frankfurt/Main 1(DE)**

(72) Erfinder: **Donnerhack, Andreas, Dr., Bismarckstrasse 4,
D-4150 Krefeld(DE)**
Erfinder: **Thoma, Klemens, Am Kleckers 22,
D-4150 Krefeld-Hüls 29(DE)**
Erfinder: **Volker, Wolfgang, Pastorsbusch 35,
D-4154 Tönisvorst 1(DE)**

ACTORUM AG

## Beschreibung

Die Erfindung betrifft eine Vorrichtung zur Durchführung der Kryotherapie am ganzen Körper mit einem kalten Behandlungsgas, nach dem Oberbegriff des Anspruches 1.

Neben der seit einigen Jahren durchgeführten lokalen Kryotherapie mit einem kalten Behandlungsgas, beispielsweise zur Behandlung rheumatischer Erkrankungen, wird bei bestimmten Krankheitsformen auch eine Kryotherapie am ganzen Körper durchgeführt. Hierbei wird mit Hilfe von flüssigem Stickstoff Luft in Wärmetauschern abgekühlt und in eine geschlossene Kammer eingedüst. Diese Kammer oder Kabine besitzt Wände aus isolierendem Material und Anschlüsse zur Zufuhr und Ableitung des Behandlungsgases. Eine solche Kammer zeigt beispielsweise das japanische Gebrauchsmuster Nr. 168 125/81. Dieses Konzept findet jedoch sowohl bei Ärzten als auch bei den Patienten wenig Anklang. Die Gründe dafür sind vielfältig. Die Patienten beanstanden den fehlenden direkten Kontakt zum Arzt, da während der Behandlung nur ein indirekter Kontakt durch Fenster und über Sprecheinrichtungen möglich ist. Die starke Nebelbildung in der Kammer verstärkt noch diesen Eindruck des mangelnden direkten Kontaktes. Nachteilig ist auch die unerwünschte Abkühlung im Kopfbereich des Patienten 7 und um ihn herum.

Außerdem muß durch besondere Maßnahmen das Einatmen kalter Luft verhindert werden. Hiervon abgesehen, erfordern solche Kammern auch hohe Investitionskosten. Wegen der langen Anlaufzeiten besteht eine Notwendigkeit zum Dauerbetrieb, wodurch verhältnismäßig hohe Betriebskosten entstehen. Die Patientenüberwachung während der Behandlung ist aufwendig.

Der Erfindung liegt daher die Aufgabe zugrunde, eine Vorrichtung zur Durchführung der Kryotherapie am ganzen Körper mit einem kalten Behandlungsgas zu schaffen, welche einen direkten Kontakt zwischen Arzt und Patienten während der Behandlung ermöglicht, den Kopf des Patienten freiläßt und wegen kurzer Anlaufzeiten keinen Dauerbetrieb erforderlich macht.

Ausgehend von dem im Oberbegriff des Anspruches berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Zwei Ausführungsbeispiele der Erfindung sollen anhand der beigefügten Zeichnungen erläutert werden.

Es zeigen:

Fig. 1 eine Vorrichtung zur Behandlung eines stehenden Patienten,

Fig. 2 eine Vorrichtung zur Behandlung eines liegenden Patienten,

Fig. 3 eine Einrichtung zur Erzeugung des Behandlungsgases, welche einen kostengünstigen Leerlaufbetrieb der Vorrichtungen ermöglicht.

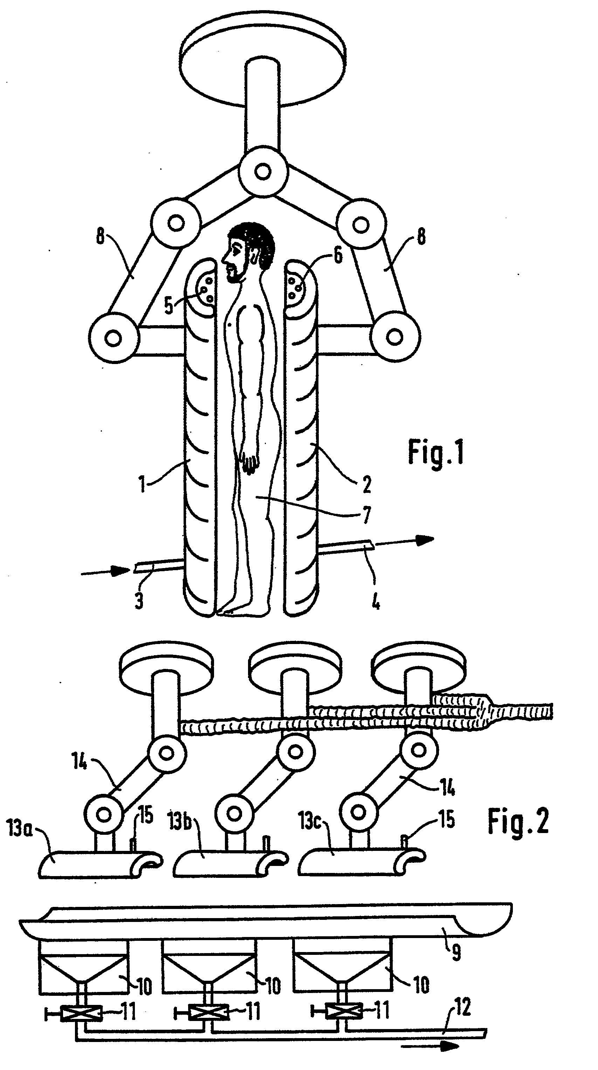

Die in Fig. 1 dargestellte Vorrichtung besteht aus zwei zylinderförmigen Halbschalen 1, 2, welche den Behandlungsraum einschließen. Das kalte Behandlungsgas tritt durch die Leitung 3 in die Schale 1 ein und wird durch die Leitung 4 aus der Schale 2 abgesaugt. Die Schalen 1, 2 sind so hoch, daß der Kopf des Patienten 7 außerhalb des Behandlungsraums bleibt. Auf den Innenseiten der Schalen 1, 2 befinden sich Öffnungen 5, 6, durch welche das Behandlungsgas in den Behandlungsraum einströmt, bzw. aus dem Behandlungsraum abgesaugt wird. Es ergibt sich somit eine im wesentlichen senkrechte Gasströmung auf den Körper des Patienten 7 und um ihn herum.

Der gegenseitige Abstand der Schalen 1, 2 kann im einfachsten Fall durch von Hand zu betätigende Riegel fixiert werden, welche die beiden Schalen 1, 2 miteinander verbinden. Eine besonders gute Raumausnutzung ermöglichen jedoch die Gelenkarme 8, die an den Schalen 1, 2 angeordnet sind und an der Decke des Klinikraumes befestigt sind. Mit Hilfe der Gelenkarme 8 kann die Stellung der Schalen 1, 2 der jeweiligen Behandlungssituation angepaßt werden.

Die Öffnungen 5 sind zweckmäßigerweise als Matrix auf der Innenseite der Schale 1 so angeordnet und dimensioniert, daß sich eine von der gesamten Fläche der gewölbten Schale 1 ausgehende gerichtete Gasströmung ergibt, wenn kaltes Behandlungsgas durch die Leitung 3 zugeführt wird. Die Absaugung des Behandlungsgases durch die Öffnungen 6 auf der Innenseite der Schale 2 und durch die Leitung 4 unterstützt die Ausbildung dieser gerichteten Gasströmung. Die gerichtete Gasströmung stellt sicher, daß der gesamte Körper mit Ausnahme des Kopfbereiches gekühlt wird.

Während Fig. 1 eine Ausführungsform der Erfindung zur Behandlung stehender Patienten zeigt, ist in Fig. 2 eine Ausführungsform der Erfindung zur Behandlung liegender Patienten dargestellt. Das Grundprinzip des aus zylinderförmigen Halbschalen gebildeten Behandlungsraumes, welcher das Gesicht des Patienten freiläßt, ist jedoch auch hier beibehalten. Die zum Absaugen des Behandlungsgases dienende Schale 9 ist hierbei als ein grobmaschiges Gitterrost ausgebildet, dessen Stege beheizbar sind. Hierauf wird der Patient gebettet. Unterhalb der Schale 9 befinden sich Absaugtrichter 10, welche über Absperrventile 11 an die Absaugleitung 12 angeschlossen sind.

Die Schale zur Zuführung des Behandlungsgases ist geteilt und besteht aus den drei Teilschalen 13a, 13b, 13c. Die Gasaustrittsöffnungen sind in diesen Teilschalen 13a, 13b, 13c so ausgebildet, wie in der Schale 1 von Fig. 1. Jede Teilschale kann mittels eines Gelenkarmes 14 zur Decke des Klinikraumes hin abgehoben werden. Die Zufuhr des kalten Behandlungsgases erfolgt durch Schläuche, welche an die Anschlüsse 15 angeschlossen werden.

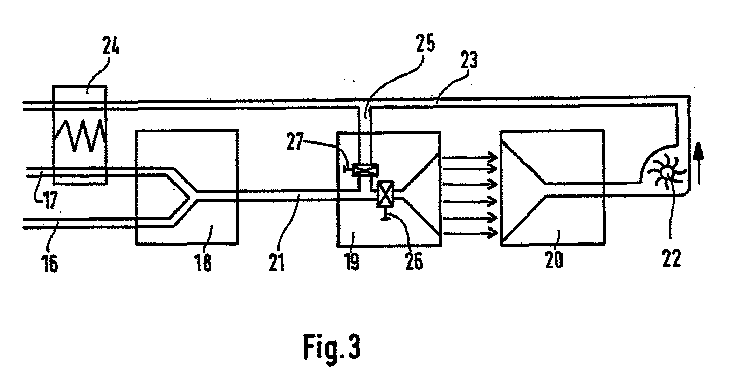

Zur Herstellung des kalten Behandlungsgases können alle hierfür gebräuchlichen Einrichtungen und Gasgemische verwendet werden. Vorteilhaft ist die Herstellung aus einem verflüssigten Gas, insbesondere Stickstoff und trockener Luft, weil sich hiermit in einfacher Weise ein kostensparender Leerlaufbetrieb der erfindungsgemäßen Vorrichtung realisieren läßt. Eine hierzu geeignete Einrichtung ist in Fig. 3 dargestellt. Durch die Leitung 16 wird flüssiger Stickstoff und durch die Leitung 17 trockene Luft in das Mischgerät 18 eingespeist. Das im Mischgerät 18 gebildete kalte Behandlungsgas strömt durch die Leitung 21 in die Eintrittsschale 19. Die Eintrittsschale 19 entspricht den Schalen 1 bzw. 13a, 13b, 13c in den Figuren 1 und 2. Das kalte Behandlungsgas durchströmt den zwischen den Schalen befindlichen Behandlungsraum mit dem Patienten und gelangt in die Austrittsschale 20, die den Schalen 2 bzw. 9 in den Figuren 1 und 2 entspricht. Aus der Austrittsschale 20 wird es durch das Absauggebläse 22 abgesaugt und mittels der Leitung 23 durch den Wärmetauscher 24 geleitet. Im Wärmetauscher 24 gibt es seine Kälte an die eintretende Luft in Leitung 17 ab. Auf diese Weise wird bei Betrieb der erfindungsgemäßen Vorrichtung die Kälte optimal ausgenutzt. Die Absaugung bewirkt eine gerichtete Strömung und dient der Kälterückgewinnung und stabilisiert die Sauerstoffkonzentration im Klinikraum.

Wenn kein Patient behandelt wird, die Vorrichtung aber betriebsbereit gehalten werden soll, wird die Anlage auf Leerlaufbetrieb umgeschaltet. Bei Leerlauf strömt das kalte Behandlungsgas unmittelbar durch die Leitung 25 in den Wärmetauscher 24 zurück. Die Umschaltung auf Leerlaufbetrieb erfolgt durch Betätigen der Ventile 26 und 27. Der Leerlaufbetrieb wird zweckmäßigerweise mit einer geringeren Gasmenge aufrechterhalten. Er soll lediglich das Kalthalten der Kaltwinderzeugungsanlage und der Zuleitungen gewährleisten. Die erfindungsgemäße Vorrichtung ist somit auch in Behandlungspausen betriebsbereit und bleibt kurzfristig einsetzbar. Ein zusätzlicher Vorteil besteht darin, daß der Patient nicht unter Kühlbedingungen positioniert werden muß.

Nicht dargestellt sind in den Figuren 1 und 2 die üblichen Kontroll- und Sicherheitseinrichtungen, insbesondere ein Sauerstoff-Sensor im Kopfbereich des Patienten, der bei Sauerstoffmangel in der Atemluft, z. B. infolge einer Störung, den Kühlbetrieb automatisch abschaltet. Zur berührungslosen Temperaturüberwachung der Hautoberfläche des Patienten dienen in die Schalen 1 und 13a bis 13c integrierte Infrarot-Sonden. Die in Fig. 2 dargestellte Ausführungsform ermöglicht auch die Teilbehandlung größerer Körperpartien eines Patienten. In einem solchen Fall wird nur die entsprechende Teilschale mit kaltem Behandlungsgas beaufschlagt. Im Gegensatz zu den bisher üblichen Kältekammern ist die Anlaufzeit bei der erfindungsgemäßen Vorrichtung extrem kurz. Es besteht daher keine Notwendigkeit zu einem kostenintensiven Dauerbetrieb. Ein direkter Sprechkontakt zwischen Patient und Arzt ist jederzeit während der Behandlung möglich. Der Kopf des Patienten bleibt frei von kaltem Behandlungsgas, weshalb die bisher üblichen aufwendigen Schutzmaßnahmen weitgehend entfallen.

**Patentansprüche**

1. Vorrichtung zur Durchführung der Kryotherapie am ganzen Körper mit einem kalten Behandlungsgas bestehend aus einer zumindest weitgehend geschlossenen Kabine zur Aufnahme des Patienten (7), welche Anschlüsse (3, 4) zur Zufuhr und Ableitung des Behandlungsgases besitzt, dadurch gekennzeichnet, daß die Kabine aus einander gegenüberliegenden, den Patienten umschließenden Schalen (1, 2, 9, 13a, 13b, 13c) gebildet wird, welche gegenüberliegend jeweils Öffnungen (5, 6) für das Einleiten bzw. Absaugen des Behandlungsgases besitzen und die das Gesicht des Patienten freilassen.

2. Vorrichtung nach Anspruch 1, gekennzeichnet durch zwei vertikal angeordnete Schalen (1, 2) zur Aufnahme eines stehenden Patienten.

3. Vorrichtung nach Anspruch 2, gekennzeichnet durch an den Schalen angeordnete Gelenkarme (8), die an der Decke des Klinikraumes befestigt sind und aus einer Ruhelage heraus ein Einschwenken der Schale in die Betriebsposition ermöglichen.

4. Vorrichtung nach Anspruch 1, gekennzeichnet durch horizontal angeordnete Schalen (9, 13a, 13b, 13c,) zur Aufnahme eines liegenden Patienten, wobei die untere Schale (9) zum Absaugen des Behandlungsgases dient.

5. Vorrichtung nach Anspruch 4, dadurch gekennzeichnet, daß die untere Schale als grobmaschiger Gitterrost mit beheizbaren Stegen ausgebildet ist.

6. Vorrichtung nach Anspruch 4 oder 5, dadurch gekennzeichnet, daß die obere Schale mehrteilig ausgeführt ist.

7. Vorrichtung nach einem der Ansprüche 4 bis 6, gekennzeichnet durch an der oberen Schale angeordnete Gelenkarme (14), die an der Decke des Klinikraumes befestigt sind und aus einer Ruhelage heraus ein Einschwenken der Schale in die Betriebsposition ermöglichen.

**Claims**

1. Apparatus for carrying out cryotherapy of the whole body using a cold treatment gas, consisting of an at least substantially closed cabin for receiving the patient (7), which cabin has connections (3, 4) for the supply and drawing off of the treatment gas, characterized in that the cabin is made up of shells (1, 2, 9, 13a, 13b, 13c) facing each other and enclosing the patient, which shells have openings (5, 6), in each case opposite each other, for the introduction and removal by suction of the treatment gas, and which leave the face of the patient free.

2. Apparatus according to Claim 1, characterized by two vertically arranged shells (1, 2) for receiving a standing patient.

3. Apparatus according to Claim 2, characterized by articulated arms (8) arranged on the shells, which arms are secured on the ceiling of the hospital room and allow the shell to be swung in from a rest position into the operating position.

4. Apparatus according to Claim 1, characterized by horizontally arranged shells (9, 13a, 13b, 13c) for receiving a patient in a lying position, the lower shell

(9) sewing for removal of the treatment gas by suction.

5. Apparatus according to Claim 4, characterized in that the lower shell is designed as a wide-meshed grid with heatable bridges.

6. Apparatus according to Claim 4 or 5, characterized in that the upper shell is designed in several parts.

7. Apparatus according to one of Claims 4 to 6, characterized by articulation arms (14) arranged on the upper shell, which arms are secured on the ceiling of the hospital room and allow the shell to be swung in from a rest position into the operating position.

**Revendications**

1. Dispositif pour l'application de la cryothérapie à l'ensemble du corps avec un gaz de traitement froid, dispositif constitué par une cabine fermée, tout au moins dans une large mesure, et destinée à recevoir le patient (7), cette cabine comportant des raccordements (3, 4) pour amener et évacuer le gaz de traitement, dispositif caractérisé en ce que la cabine est constituée par des coquilles (1, 2, 9, 13a, 13b, 13c) placées les unes en face des autres, et entourant le patient, ces coquilles comportant respectivement des orifices placés les uns en face des autres (5, 6) pour l'introduction ou bien l'aspiration du gaz de traitement, et ces coquilles laissant libre le visage du patient.

2. Dispositif selon la revendication 1, caractérisé en ce qu'il comporte deux coquilles disposées verticalement (1, 2) pour recevoir un patient debout.

3. Dispositif selon la revendication 2, caractérisé en ce qu'il comporte des bras articulés (8) disposés sur les coquilles, ces bras étant fixés au plafond du local de la clinique et permettant à partir d'une position de repos, d'amener les coquilles dans la position de fonctionnement.

4. Dispositif selon la revendication 1, caractérisé en ce qu'il comporte des coquilles (9, 13a, 13b, 13c) disposées horizontalement et destinées à recevoir un patient allongé, la coquille inférieure (9) étant utilisée pour l'aspiration du gaz de traitement.

5. Dispositif selon la revendication 4, caractérisé en ce que la coquille inférieure revêt la forme d'un caillebotis à larges mailles avec des nervures susceptibles d'être chauffées.

6. Dispositif selon la revendication 4 ou 5, caractérisé en ce que la coquille supérieure est réalisée en plusieurs parties.

7. Dispositif selon une des revendications 4 à 6, caractérisé en ce qu'il comporte des bras articulés (14) disposés sur les coquilles supérieures, ces bras étant fixés au plafond du local de la clinique et permettant d'amener, à partir d'une position de repos, les coquilles dans la position de fonctionnement.

EP 0 220 478 B1

Fig.1

Fig.2

Fig.3